# EUROPEAN PATENT APPLICATION

(11) **EP 1 324 043 A1**
(43) Date of publication of application: **02.07.2003**
(21) Application number: 01956924.3
(22) Date of filing: 16.08.2001
(51) Int. Cl.: G01N 33/547, G01N 33/543, G01N 33/566, G01N 33/53

(54) **SOLID SUPPORTS HAVING SURFACE-TREATED LAYER FORMED THEREON**

(30) Priority: 06.09.2000 JP 2000270775; 27.06.2001 JP 2001195573
(71) Applicant: Toyo Kohan Co., Ltd., Tokyo 102-8447 (JP)
(72) Inventor: TANGA, Michifumi, Kudamatsu-shi, Yamaguchi 744-8611 (JP); OKAYAMA, Hironao, Kudamatsu-shi, Yamaguchi 744-8611 (JP); OKAMURA, Hiroshi, Kudamatsu-shi, Yamaguchi 744-8611 (JP); EHARA, Keigo, Kudamatsu-shi, Yamaguchi 744-8611 (JP); TAKAGI, Kenichi, Kudamatsu-shi, Yamaguchi 744-8611 (JP)
(74) Representative: VOSSIUS & PARTNER
(86) International application number: JP0107065
(87) International publication number: WO02021131

(57) **Abstract**

For the purpose of overcoming a conventional art problem of spot detachment during the process of gene analysis (for example, hybridization) by strongly immobilizing biological material samples such as DNA or protein via covalent bonding on a substrate, the invention provides a solid support with a surface-treated layer of hafnium carbide, niob carbide, silicone carbide, tantalum carbide, thorium carbide, titanium carbide, uranium carbide, tungsten carbide or zirconium carbide as formed on the surface thereof so as to enable the immobilization of oligonucleotide or DNA fragment on the surface thereof; a solid support immobilizing oligonucleotide or DNA fragment on the film of the surface-treated layer; and a method for analyzing gene by immobilizing gene on the surface of such solid support.

## Description

### Technical Field

The present invention relates to a solid support for use in the analysis of biological materials such as gene and protein to be used for gene analysis, diagnosis and therapeutic treatment, and a method for analyzing biological materials such as gene or protein, using the solid support.

### Background of the Invention

Solid supports such as glass chip with processed surface so as to mount gene such as 10,000 or more DNA fragments (DNA probes) thereon have widely been used so far as solid supports for use in gene analysis.

In case that it is intended to elucidate the nucleotide sequence of a certain DNA sample, using chips as described above, for example, preparing several tens of thousands of DNA fragments of which the nucleotide sequences are preliminarily elucidated and are different from each other are bound to the solid support so that their positions can be identified and then allowing a DNA sample labeled with fluorescence to flow on the resulting solid support, a DNA fragment in the DNA sample hybridizes to a probe with a complementary sequence, among the DNA fragments (DNA probes) bound to the solid support. The hybridizing site can be identified in the form of spot, by assaying the fluorescence on the solid support. Thus, the sequence of the DNA fragment in the DNA sample can be elucidated.

As described above, the nucleotide sequence of a certain DNA can be identified readily on such solid support for gene analysis. Therefore, such solid support can be used for the analysis of biological genome, the monitoring of gene expression, and gene analysis such as genome mismatching and can additionally be applied to gene diagnosis such as the detection of oncogene mutation and the development of pharmaceutical product.

Allowing a DNA sample labeled with fluorescence to hybridize using the solid support for gene analysis and . subsequently allowing fluorescent irradiation on the solid support thereby analyzing the resulting spot, the DNA sample can be assayed.

Because conventional solid supports for gene analysis require pre-treatment such as glass rinsing for the spot analysis, however, the DNA fragment on the spot is rinsed off, leading to no clear detection of the spot.

It is an object of the invention to overcome such ambiguousness in the detection of fluorescence on the conventional solid supports for gene analysis.

### Disclosure of the Invention

In accordance with the invention, it has been found that by forming a surface-treated layer on a solid support such as glass, plastic and silicone as the substrate and further implementing chemical modification of the solid support, the resulting substrate can strongly immobilize spotted DNA fragments with no rinsing off of the DNA fragments even after the solid support is rinsed. Additionally, the fluorescent spot after the irradiation of the fluorescence is sharper. The invention is based on such finding.

The 'solid support of the invention includes a surface-treated layer of hafnium carbide, niob carbide, silicone carbide, tantalum carbide, thorium carbide, titanium carbide, uranium carbide, tungsten carbide, zirconium carbide, molybdenum carbide, chromium carbide and vanadium carbide on the surface.

The thickness of the film of the surface-treated layer is preferably 1 nm to 1,000 nm.

Additionally, preferably, the film of the surface-treated layer is preferably chemically modified and immobilized with oligonucleotide or DNA fragment thereon.

As recited in claim 5, such solid support of the invention can be used for the method for analyzing biological materials such as gene or protein, by immobilizing gene on the surface of the solid support.

### Brief Description of the Drawing

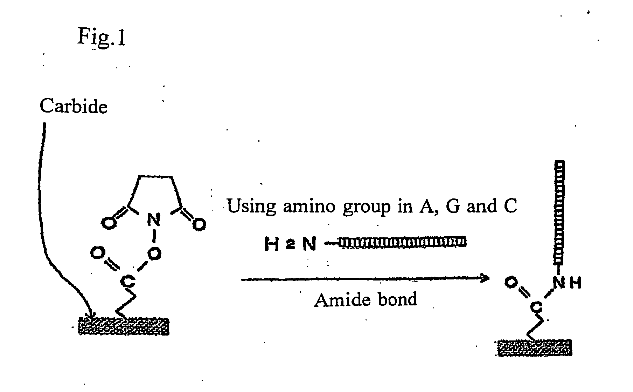

Fig. 1 is a schematic explanatory view of the immobilization of probe on the solid support.

### Best Mode for Carrying out the Invention

When a solid support such as glass, plastic and silicone with an appropriate surface-treated layer formed on the top surface thereof is used as the solid support of the invention for various analyses while DNA samples are mounted on the solid support, the affinity to biological materials such as gene and protein is elevated preferably.

Any known plastic can be used as the plastic as the solid support. For example, thermosetting or thermoplastic resins can be used, including polyester resin such as polyethylene terephthalate or polybutylene terephthalate, polyethylene resin, polystyrene resin, polypropylene resin, ABS resin, nylon, acryl resin, fluorine resin, polycarbonate resin, polyurethane resin, methylpentene resin, phenol resin, melamine resin, epoxy resin and vinyl chloride resin.

For surface treatment, further, carbides such as hafnium carbide, niob carbide, silicone carbide, tantalum carbide, thorium carbide, titanium carbide, uranium carbide, tungsten carbide, zirconium carbide, molybdenum carbide, chromium carbide and vanadium carbide are preferably coated on the surface.

Furthermore, mixtures or laminates of the above carbides with other materials, such as metals and ceramics, are also preferable.

In other words, carbon is great in terms of chemical stability and can endure subsequent chemical modification or reaction during the mounting of DNA probes and the like.

The reason may be as follows: when carbides are chemically modified for immobilization of a probe, the probe takes the binding form to the carbon in the carbides as shown in Fig.1, so that the DNA probe can be immobilized strongly on the solid support.

Because the immobilized probe can be arranged vertically in an array on the solid support as shown in Fig. 1, additionally, the immobilization density per unit area can be elevated.

The thickness of the surface-treated layer of the carbide in accordance with the invention is satisfactorily 1 nm to 1,000 nm with no specific limitation. Below 1 nm, unpreferably, the surface-treated layer is too thin so that the thickness of the surface-treated layer is not uniform, involving a part where the underlining solid support is exposed. At the film thickness above 1,000 nm, in contrast, stress occurs in the surface-treated layer during the formation, leading to ready occurrence of stripping, unpreferably. From the standpoint of industrial-production, preferably, the thickness of the surface-treated layer is 10 nm to 500 nm. More preferably, the thickness thereof is 30 to 200 nm.

As the method for forming the surface-treated layer of the carbides on the solid support, known methods can be used. The methods include for example high frequency sputtering, direct current sputtering, arc ion plating, and thermal CVD method.

The solid support of the invention can mount a great number of biological materials such as gene including DNA probe or protein thereon. Thus, a solid support with extremely small plural fractions arranged on the surface thereof to immobilize numerous oligonucleotide fragments on each fraction is preferably used. On each of the extremely small fractions may be arranged a different type of DNA probe and the like, with no specific limitation. Depending on the use, appropriately, the DNA probe can be modified.

The form of the solid support includes but is not limited to for example plate-like forms such as those of film or sheet or disc-like forms. Further, the thickness and dimension of the solid support are not specifically limited but can be within ranges for general use.

The characteristic properties of glass as the substrate of the solid support are not specifically limited. Glass with appropriate characteristic properties can be selected, taking account of various properties such as the affinity to reactants attached on the substrate surface.

On the surface or back face of the substrate may additionally be arranged a single layer of Ti, Au, Pt, Nb or WC or a complex film thereof as the reflection layer. The thickness of the reflection layer is preferably 100 nm or more, because such reflection layer covers the entirety uniformly. More preferably, the thickness thereof is 1000 nm or more.

Preferably, furthermore, the surface of the underlining solid support is intentionally modified into rough surface. Owing to such rough surface, the substrate can have increased surface area, advantageously, for the immobilizing of a great number of DNA probes and the like, due to the increase of the density.

So as to immobilize DNA and protein on the surface of the substrate, the surface is further chemically modified. One example of the chemical modification is immobilization of a hydrocarbon group at its end bound with an activated ester group through amide bond, on the support surface. Via such chemical modification, biological materials such as DNA, protein and peptide bond can more readily be immobilized on the surface of the substrate. The chemical modification serves for the substitution of the solid support with hydrocarbon groups with polar groups at their ends, for example hydroxyl group carboxyl group, epoxy group, amino group, thiol group, and isocyanate group.

The above hydrocarbon groups are preferably hydrocarbon groups with one to 12 carbon atoms, preferably one to 6 carbon atoms. The hydrocarbon groups include for example monocarboxylic acids such as formic acid, acetic acid and propionic acid; dicarboxylic acids such as oxalic acid, malonic acid, succinic acid, maleic acid and fumaric acid; and polycarboxylic acids such as trimellitic acid. Among them, oxalic acid and succinic acid are preferable.

The chemical modification method includes a step of allowing ultraviolet ray to irradiate the support in chlorine gas to chlorinate the surface, a step of subsequently allowing ultraviolet ray to irradiate the support in ammonia gas for amination of the support and a step of carboxylating the surface using an appropriate acid chloride or acid anhydride.

The oligonucleotide or DNA fragment (probe) to be mounted on the solid support of the invention includes for example any single stranded or double-stranded DNA- and RNA fragments with any number of the nucleotides with no specific limitation. The oligonucleotide or DNA fragment can be immobilized via chemical bonding and the like on the surface of the solid support. In case of using a solid support with a formed surface-treated layer of a carbide, for example, the surface is activated, i.e. the surface is modified for ready chemical bonding to DNA, and is then bonded to the amino group in the terminal nucleotide of DNA.

One example of the chemical modification or activation of the solid support with the formed surface-treated layer in this case is as follows. Allowing ultraviolet ray to irradiate the solid support in chlorine gas to chlorinate carbon in the carbide and to subsequently irradiate the solid support in ammonia gas for amination, implementing carboxylation using an appropriate acid chloride, and allowing the terminal carboxyl group and N-hydroxysuccinimide to undergo dehydration condensation, using a dehydration condensation agent carbodiimide, dicyclohexylcarbodiimide, or 1-[3-(dimethylamino)propyl] 3-ethylcarbodimide, a group bound with an active ester group such as N-hydroxysuccinimide ester group via amide bond at the end of a hydrocarbon group can be immobilized, so that the surface-treated layer can be activated.

When the surface of the solid support of the invention is activated in such manner, for example, several tens of thousands of DNA fragments (probes) with the elucidated nucleotide sequences can be immobilized on the surface.

Additionally, once oligodT primer is attached on the solid support, an objective cDNA is extended via reverse transcription reaction and is simultaneously immobilized on the solid support.

Using PCR and the like, further, numerous DNA chains can be extended and immobilized on the solid support.

When a fluorescence-labeled DNA sample flows on the solid support after DNA fragments are immobilized in such manner, the DNA sample hybridizes to a probe with a complementary sequence among the DNA fragments (probes) immobilized on the solid support. Then, the sequence of the DNA sample can be identified in the form of fluorescent spot.

In such manner, the solid support of the invention enables analysis and identification of the nucleotide sequence of a certain DNA, far more clearly than conventional art does, using conventional art methods as they are. Therefore, the solid support can be used for the analysis of biological genome, the monitoring of gene expression, the gene analysis of genome mismatching and the like. Additionally, the solid support is useful for gene diagnosis such as the detection of oncogene mutation as well as for the development of pharmaceutical product.

### Examples

The invention is now described in more detail in the following examples.

### Example 1

1. A polyethylene terephthalate resin as a solid support for use in gene analysis was prepared as described below. First, a polyethylene terephthalate resin of 25 mm (width) × 75 mm (length) × 1 mm (thickness) was used. Using target carbides such as hafnium carbide, niob carbide, silicone carbide, tantalum carbide, titanium carbide, tungsten carbide and zirconium carbide, then, films of the individual target carbides were prepared at a thickness of about 10 nm on the surface of the polyethylene terephthalate resin, by high frequency sputtering using argon gas as working gas, so that solid supports were prepared.
2. Then, the surface of these solid supports was chemically modified for activation.

Specifically, the surface of the polyethylene terephthalate resin was chlorinated for one minute, subsequently aminated for 10 minutes and directly immersed in succinyl chloride for 10 minutes. Then, the solid supports were rinsed with ultra pure water, and subsequently immersed in an activation solution for direct activation. The activation solution was of a composition of 1 mL of 1, 4-dioxane, 25 mg of hydrogen cyanamide, and 150 mg of N-hydroxysuccinimide in dissolution. The resulting solid supports were further rinsed with ultra pure water and dried at 65 °C, for activation. 2µL of FAMdA17 solution at a concentration of 500 pmol/mL was dropwise added (spotted) onto the surface of the solid supports as prepared as described above. As buffers, in this case, ultra pure water or 10 % formamide, 10 % glycerin, or 50 % DMSO was used.

Subsequently, incubation was done. The conditions were 65 °C in the atmosphere water/formamide = 1/1 for one hour (drying).

The fluorescence intensity of the solid supports thus recovered for use in gene analysis was measured. The time for the measurement was one minute, using an apparatus LAS-1000 Plus. After spotting and after drying (65 °C), the fluorescence intensity was measured. All the resulting intensities were superior to those recovered on the conventional art solid supports.

All the solid supports of the invention show greater fluorescence intensities after spotting and after drying than those of conventional art solid supports after spotting and after drying. In other words, fragments of biological materials, such as spotted DNA or protein were never rinsed off from any of the solid supports of the invention but remained thereon, in any case, so that the spots could be detected clearly.

Alternatively, the fragments of biological materials, such as spotted DNA or protein were rinsed off from the conventional art solid support and never remained thereon, in any case, so that the spot could not be detected clearly.

### Example 2

As a substrate, silicone of 25 mm (width) × 75 mm (length) × 0.5 mm (thickness) was used. On a surface of the silicone substrate, using a target tantalum carbide then, a film of the carbide was prepared at a thickness of about 10 nm by high frequency sputtering using argon gas as working gas.

Then, the surface of the silicone substrate was chemically modified, for activation. The surface of the silicone substrate was chlorinated under ultraviolet irradiation in chlorine gas for one minute, subsequently aminated in ammonia gas for 10 minutes and directly immersed in a succinic anhydride solution for 20 minutes. The succinic anhydride solution was prepared, by dissolving succinic anhydride and sodium borate (pH8) to 140 mM/L and 0.1 M/L, respectively in N-methyl-2-pyrrolidone.

Then, the silicone substrate was directly activated via immersion in an activation solution. The activation solution was prepared, by dissolving 115 mg of N-hydroxysuccinimide and 959 mg of 1-[3-(dimethylamino)propyl] 3-ethylcarbodiimide in 50 mL of phosphate buffer (pH6) in a 200-mL beaker. The silicone substrate was immersed in the activation solution, for reaction. Then, the resulting silicone substrate was rinsed.

DNA was spotted on the activated substrate. A spotting solution was prepared by dissolving a DNA sample in 50 % DMSO solution (spotting buffer) to a concentration of 0.3 µg/µL. Using then a spotting apparatus, the DNA solution was pressed against a slide glass. In such manner, a great number of DNA samples were attached on the surface of the slide glass.

Subsequently, the slide glass was incubated for DNA immobilization. First, a solution of water and formaldehyde mixed together at 1:1 was placed in a tight box, which was a chamber at an adjusted humidity. Then, the silicone substrate was placed in the humidity-adjusted chamber while avoiding the contact to the solution. Then, the silicone substrate was left therein for 3 hours. Subsequently, the silicone substrate was rinsed twice with rinse solutions (2× SSC, 0.2 % SDS) and additionally rinsed with 0.1 % SSC and sterile water, for centrifugation and drying.

Then, the silicone substrate was blocked with a prehybridization solution (50 % formamide, 5 × Denhardt solution). The blocking solution was dropwise added to the substrate, on which a cover glass was gently mounted so as to avoid the infiltration of gas. Subsequently, the substrate was placed in the humidity-adjusted chamber, for one-hour blocking at 60 °C. Then, the cover glass was rinsed off with 0.1 × SSC, followed by rinsing with sterile water for 15 minutes and subsequent centrifugation and drying.

Thereafter, a DNA sample was labeled with fluorescence, using a fluorescence labeling kit. Then, the labeled DNA was denatured in alkaline. The label used for labeling was dissolved in a hybridization buffer (20 % SSC, 20 % formamide, 0.5 % SDS), to adjust the concentration to 0.3 µg/µl. The resulting solution was defined as hybridization solution.

After the silicone substrate with immobilized DNA thereon was immersed in hot water for 5 minutes, the hybridization solution was dropwise added on the substrate, on which a cover glass was gently mounted so as to avoid the infiltration of gas. Subsequently, the DNA was allowed to hybridize in the humidity-adjusted chamber for 12 hours.

Thereafter, 0.1 × SSC was used for washing off the cover glass. The silicone substrate was twice rinsed with rinsing solutions (2× SSC, 0.2 % SDS) and additionally rinsed with 0.1 % SSC and sterile water, for centrifugation and drying.

The resulting substrate was observed with a Fuji Film fluoroimage analyzer FLA-8000. In accordance with the invention, the fluorescence intensity was 1352, higher than the value 845 in the conventional art.

### Industrial Applicability

The solid support of the invention enables analysis and identification of the nucleotide sequence of a certain DNA far more clearly than conventional art does, using conventional art methods as they are. Therefore, the solid support can be used for analysis of biological materials such as gene or protein, including the analysis of biological genome, the monitoring of gene expression and the gene analysis of genome mismatching. Additionally, the solid support is useful for gene diagnosis such as the detection of oncogene mutation as well as for the development of pharmaceutical product.

## Claims

1. A solid support with a surface-treated layer of hafnium carbide, niob carbide, silicone carbide, tantalum carbide, thorium carbide, titanium carbide, uranium carbide, tungsten carbide, zirconium carbide, molybdenum carbide, chromium carbide and vanadium carbide formed on the surface thereof.

2. A solid support according to claim 1, where the thickness of the surface-treated layer is 1 nm to 1,000 nm.

3. A solid support according to claim 1 or 2, where oligonucleotide or DNA fragment is immobilized on the film on the surface-treated layer.

4. A method for analyzing biological materials such as gene or protein, by immobilizing gene on the surface of a solid support according to any one of claims 1 to 3.
